# EUROPÄISCHE PATENTSCHRIFT

(11) **EP 0 916 647 B1**
(45) Veröffentlichungstag und Bekanntmachung des Hinweises auf die Patenterteilung: **05.01.2005**
(21) Anmeldenummer: 98120724.4
(22) Anmeldetag: 31.10.1998
(51) Int. Cl.: C07C 229/14, C08G 18/08, C08G 18/44, C08G 18/48, C08G 18/80, C09D 175/04

(54) **Verwendung eines Hydrophilierungsmittels als Dispergator für wässrige Polyurethan-Dispersionen**
Use of a hydrophilic agent as dispersing agent for aqueous polyurethane dispersions
Utilisation d'un agent hydrophile comme agent dispersant pour des dispersions aqueuses de polyuréthane

(30) Priorität: 13.11.1997 DE 19750186
(43) Veröffentlichungstag der Anmeldung: 19.05.1999
(73) Patentinhaber: Bayer MaterialScience AG, 51368 Leverkusen (DE)
(72) Erfinder: König, Eberhard, Dr., 51375 Leverkusen (DE); Schütze, Detlef-Ingo, Dr., 51061 Köln (DE); Meixner, Jürgen, Dr., 47803 Krefeld (DE)

(56) Entgegenhaltungen:
- EP-A- 0 000 568
- EP-A- 0 269 972
- EP-A- 0 312 890
- DE-A- 2 034 479
- DE-A- 3 139 966
- US-A- 5 008 325
- DATABASE CHEMLIST [Online] STN; RN: 84540-26-1; EINECS No. 283-118-17, 1990 Database accession no. 136399 XP002209977 & ANNEX TO OFFICIAL JOURNAL OF THE EUROPEAN COMMUNITIES, C146A, 15. Juni 1990 (1990-06-15),
- DATABASE WPI Section Ch, Week 198049 Derwent Publications Ltd., London, GB; Class A18, AN 1980-87149C XP002226440 & JP 55 135142 A (MATSUMOTO YUSHI SEIYAKU KK), 21. Oktober 1980 (1980-10-21)

## Beschreibung

Die vorliegende Erfindung betrifft die Verwendung eines Hydrophilierungsmittels, insbesondere als Dispergator in wäßrigen PUR-Dispersionen.

Bekanntlich werden NCO-Prepolymere durch Umsetzung mit geeigneten Molekülen, die ionische Gruppen wie z.B. Carboxylatanionen tragen, hydrophiliert. Derart hydrophilierte Polyurethane können in in Wasser dispergierte Polyurethan-Kunststoffe zwecks Herstellung von Beschichtungen für z.B. Textilien, Bleche, Stein oder Holz überführt werden. Ein Review-Artikel von Rosthauser und Nachtkamp in Advances in Urethane Science und Technology", Band 10, Seite 121-162 (1987) umfaßt das Wissen auf diesem Gebiet.

Eine Gruppe von leicht zugänglichen Dispergator-Bausteinen stellen die Umsetzungsprodukte von Alkalimetallsalzen der Acrylsäure mit Diaminen dar, wie sie in der DE-A 2 034 479 beschrieben werden.

Diese Gruppe von Dispergatoren hat den Nachteil, daß durch die Anwesenheit des nichtflüchtigen Basenanteils, im obigen Falle Natrium, die Dispergatoren bleibend hydrophiliert sind, also auch nach Applikation und Einbrennen der PUR-Dispersion. Die hiermit hergestellten Textilbeschichtungen oder Lacke besitzen immer eine erhöhte Empfindlichkeit gegenüber Wasser. Ein weiterer Nachteil dieser Gruppe von Dispergatoren ist die starke gelb-braune Eigenfärbung, die sich störend bei hellfarbigen Beschichtungen bemerkbar macht.

Aufgabe der Erfindung war es daher, nach einem einfachen Verfahren einen Dispergator zur Verfügung zu stellen, der zum einen farblos ist und der zum anderen die Möglichkeit bietet, die Neutralisation, also die Salzbildung, mit einem Amin oder einem Alkalihydroxid vorzunehmen. Diese Aufgabe wurde mit dem Auffinden der erfindungsgemäßen Verbindung gelöst.

Gegenstand der Erfindung sind Hydrophilierungsmittel hergestellt durch Michael-Addition von 1-Amino-3,3,5-trimethyl-5-aminomethyl-cyclohexan mit 1 bis 2 Mol einer α,β, ungesättigten Carbonsäure und gegebenenfalls anschließende Neutralisierung mit bis zu der COOH-Gruppe äquivalenten Menge (Erd)alkalihydroxid und/oder Aminen und/oder Ammoniak.

Gegenstand der Erfindung ist schließlich die Verwendung der erfindungsgemäßen Hydrophilierungsmittel als Kettenverlängerer von NCO-Prepolymeren gegebenenfalls nach Neutralisierung der Carboxylgruppen mit Metallbasen (Erd)alkalihydroxid), oder Aminen und/oder Ammoniak zur Herstellung wäßriger PUR-Dispersionen oder hydrophilierter PUR-Beschichtungen aus Lösung für Beschichtungen von Leder und Textilien.

Erfindungswesentlich ist die Tatsache, daß die α,β-ungesättigte Säure unmittelbar und nicht ihr Alkalisalz, wie in der bereits zitierten DE-A 203 4479 beschrieben, mit Isophorondiamin umgesetzt wird und daß dieses Umsetzungsprodukt eine lagerstabile, fast farblose Verbindung ergibt. Diese lagerstabile Verbindung kann in NCO-Prepolymerketten eingebaut werden und anschließend neutralisiert werden.

Die erfindungsgemäßen Hydrophilierungsmittel stellen Umsetzungsprodukte (Additionsprodukte) von Isophorondiamin (1-Amino-3,3,5-trimethyl-5-aminomethyl-cyclohexan, IPDA) mit 1 bis 2 Mol α,β-ungesättigten Carbonsäuren, (wie z.B. Acrylsäure, Methacrylsäure oder Crotonsäure) dar. Bevorzugt ist jedoch das Additionsprodukt von IPDA und Acrylsäure, wobei ein Verbindungsgemisch aus nicht umgesetztem IPDA, Monoaddukt und Diaddukt entsteht.

Das weitaus überwiegend gebildete Monoaddukt hat die Formel 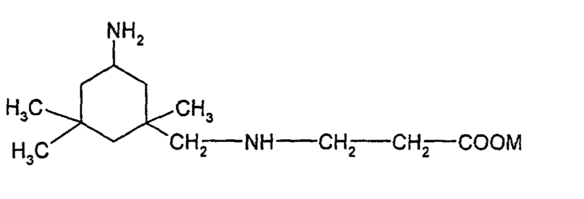 in welcher
- M: für H, (Erd)alkali wie Na, K, ^{1/2}Ca, ^{1/2}Mg oder einen aminischen Rest steht.

Als aminische Reste seien genannt: NH₄⁺, -N(R)₃ mit R = Methyl, Ethyl und/oder Hydroxyether.

Bevorzugte Amine zur Neutralisation der erfindungsgemäßen Hydrophilierungsmittel sind zum einen flüchtige tert.-Amine, wie z.B. Dimethylethanolamin oder Triethylamin, und zum anderen diprimäre Amine, die eingebaut werden können, wie z.B. Isophorondiamin oder 4,4'-Diaminodicyclohexylmethan.

Die erfindungsgemäßen Hydrophilierungsmittel können z.B. folgendermaßen hergestellt werden.

Es wird 1 Mol IPDA, bevorzugt in Wasser oder Isopropanol, vorgelegt. Unter Rühren wird hierzu bei Raumtemperatur die α,β-ungesättigte Carbonsäure, z.B. Acrylsäure, hinzugetropft. Die Umsetzung deutet sich durch eine leichte Exothermie an. Nach der Säurezugabe wird bei 40°C für 1 Stunde nachgerührt. Danach kann diese klare und farblose Lösung des Hydrophilierungsmittels eingesetzt werden. Beispielsweise kann man diese Lösung in Form des berechneten Anteils zu der acetonischen NCO-Prepolymerlösung bei ca. 40°C geben, so daß das erfindungsgemäße Hydrophilierungsmittel in die PUR-Ketten eingebaut wird. Anschließend wird mit der berechneten Menge einer Base neutralisiert. Hierbei hat man es in der Hand, ob eine bleibende Hydrophilierung, also eine Neutralisation mit nichtflüchtigen Metallhydroxiden, oder nur eine temporäre Hydrophilierung, also eine Neutralisation mit Aminen gewünscht wird. Anschließend wird die acetonische PUR-Lösung in Wasser dispergiert und, wie bekannt, durch destillative Entfernung des Acetons zu einer wäßrigen PUR-Dispersion für Beschichtungen weiterverarbeitet.

Wie teilweise bereits erwähnt, verfügen die erfindungsgemäßen Hydrophilierungsmittel über folgende vorteilhaften Eigenschaften:
- leichte Herstellbarkeit
- keine Gelbfärbung, statt dessen Farblosigkeit
- Wahlmöglichkeit der Salzbildung mit nichtflüchtigen Basen (Metallhydroxide) oder mit flüchtigen Basen (Amine)
- guteVerträglichkeit mit NCO-Prepolymeren
- Erweiterung des Angebots an carboxylathaltigen Dispergatoren.

Zu den beiden letzten Punkten folgende Erläuterung: Carboxylathaltige Hydrophilierungsmittel besitzen eine schwächere bleibende Hydrophilie als sulfonathaltige Hydrophilierungsmitel, weshalb man erstere auch für wäßrige Lackanwendungen oder Textilbeschichtungen mit hohem Eigenschaftsniveau bevorzugt. Auf dem Markt sind aber nur wenige derartige Hydrophilierungsbausteine, wie z.B. Dimethylolpropionsäure vorhanden. Die erfindungsgemäßen Hydrophilierungsmittel ergänzen das diesbezügliche Angebot und erweitern die chemischen Kombinationsmöglichkeiten, weil die neuen Hydrophilierungskomponenten auf dem bekanntermaßen gut verträglichen IPDA-Hartsegment basieren. Sie eigner sich daher sehr gut zur Beschichtung von Oberflächen der unterschiedlichsten Werkstoffe und Materialien z.B. können mit ihnen Textilwerkstoffe, Papier, Kunststoffe, Metalle, Glas usw. beschichtet werden.

### Beispiele

### Beispiel 1

### Ansatz:

| | | |
|---|---|---|
| 85,0 g | (1 Val NH₂)* | Isophorondiamin |
| 36,0 g | (0,5 Mol) | Acrylsäure |
| 282,0 g | | Wasser |
| 403,0 g | (1 Val NH/NH₂) | Dispergatorlösung |
| | | Festkörper: Ber. 30% |
| | | 1 Säureäquivalent: 806 g |

| | | |
|---|---|---|
| * bezogen auf C=C-Doppelbindung | | |

### Durchführung:

IPDA und Wasser werden bei Raumtemperatur vorgelegt. Hierzu tropft man unter Rühren Acrylsäure hinzu, wobei sich der Ansatz leicht erwärmt. Nach beendeter Acrylsäurezugabe wird 1 Stunde bei 45°C nachgerührt. Man erhält eine klare, farblose Dispergatorlösung (30%ig) mit einen NH/NH₂-Äquivalent von 403 g und einem Säureäquivalent von 806 g.

Diese Dispergatorlösung kann auch ohne weiteres in konzentrierter Form, beispielsweise 50 oder 60%ig, hergestellt werden. Es besteht dann aber die Gefahr des Auskristallisierens bei Raumtemperatur. Lagert man diese festkörperreichen Varianten jedoch bei 60 -70°C, so bleiben sie auch flüssig.

### Beispiel 2 (Anwendungsbeispiel)

### Ansatz:

| | | |
|---|---|---|
| 235,0 g | (0,236 Val) | eines Polycarbonates basierend auf Hexandiol-1,6 mit der OH-Zahl 56 |
| 80,0 g | (0,080 Val) | eines Polytetramethylenglykols mit der OH-Zahl 56 |
| 10,0 g | (0,020 Val) | eines monofunktionellen Ethylenoxidpolyethers mit Molekulargewicht 500 |
| 4,3 g | (0,064 Val) | Dimethylpropionsäure |
| 33,6 g | (0,400 Val) | 1,6-Diisocyanatohexan (HDI) |
| 35,6 g | (0,320 Val) | 1-Isocyanato-3,3,5-trimethyl-5-isocyanomethyl-cyclohexan (IPDI) |
| 28,2 g | (0,070 Val) | Dispergator gemäß Beispiel 1 |
| 7,65 g | (0,090 Val) | 1-Amino-3,3,5-trimethyl-5-aminomethylcyclohexan (IPDA) |
| 8,08 g | (0,08 Val) | Triethylamin |
| 612,0 g | | Wasser |
| 1055,43 g | | Dispersion (40%ig) |
| | | Viskosität (23°C) ca. 900 mPas |

### Durchführung:

Die obigen Polyole werden bei 100°C und 40 mbar 30 min. lang entwässert und auf ca. 90°C abgekühlt. Man gibt unter Rühren in einem Guß die beiden Diisocyanate hinzu und setzt für ca. 2 Stunden bei 90°C um. Man mißt einen NCO-Gehalt von 3,2%, berechnet sind 3,36%. Man verdünnt mit 400 g Aceton tropft die Mischung aus wäßriger Dispergatorlösung, IPDA und Triethylamin in die ca. 45°C warme acetonische NCO-Prepolymerlösung. Danach wird noch ca. 1 Stunde bei 45°C nachgerührt und mit Wasser dispergiert. Nach dem Abdestillieren des Acetons (45°C/100 mbar) erhält man eine milchig-blaue Dispersion mit einem Festkörpergehalt von ca. 40% und einer Viskosität (23°C) von ca. 900 mPas.

Die Dispersion hat als Lederbeschichtung gut Knickechtheiten, insbesondere bei Kälteknickungen (über 10 000 bei -10°C und -30°C).

### Beispiel 3

Dieses Beispiel beschreibt die Herstellung des erfindungsgemäßen Hydrophilierungsmittels in einem organischen Lösemittel.

### Ansatz:

| | | |
|---|---|---|
| 170,0 g | (2,0 Val) | Isophorondiamin |
| 72,0 g | (1,0 Mol) | Acrylsäure |
| 358,0 g | | Isopropanol |
| 600,0 g | (2,0 Val NH/NH₂) | Dispergatorlösung |
| | | Festkörper: Ber. ca. 40% |
| | | 1 Säureäquivalent: Ber. 600 g |

### Durchführung:

Isophorondiamin und Isopropanol werden vorgelegt und auf 40°C erwärmt. Hierzu tropft man unter Rühren Acrylsäure, wobei man eine geringe Exothermie beobachtet. Nach der Acrylsäure-Zugabe wird 2 Stunden bei 45°C nachgerührt. Man erhält eine anwendungsfertige farblose Dispergatorlösung mit einem NH/NH₂-Äquivalent von 300 g und einem Säureäquivalent von 600 g.

### Beispiel 4 (erfindungsgemäß)

Im Unterschied zu Beispiel 1 liegt hier eine 10 %ige Neutralisierung der Carboxylgruppen mit KOH vor.

### Ansatz:

| | | |
|---|---|---|
| 85,0 g | (1 Val NH₂) | Isophorondiamin |
| 36,0 g | (0,5 Mol) | Acrylsäure |
| 282,2 g | | Wasser |
| 2,8 g | (0,05 Mol) | Kaliumhydroxid |
| 406,0 g | (1,0 Val NH/NH₂) | Dispergatorlösung |
| | | Festkörper: Ber. ca. 30,1 % |
| | | 1 Säureäquivalent: 902 g |

### Durchführung:

Man arbeitet wie in Beispiel 1 beschrieben und rührt nach beendeter Umsetzung noch die angegebene Menge an festem Kaliumhydroxid ein. Man erhält eine klare, farblose Dispergatorlösung (ca. 30 %ig), die bereits zu 10 Äquivalent-% bleibende Salzgruppen und noch 90 Äquivalent-% nicht neutralisierte Carboxylgruppen enthält. Das Äquivalentgewicht bezogen auf die einbaubaren NH bzw. NH₂-Gruppen beträgt 406 g.

### Beispiel 5 (Anwendungsbeispiel)

In diesem Beispiel erfolgt die Neutralisierung des Dispergators zu 30 Äquivalent-% mit 4,4'-Diamino-dicyclohexylmethan, das beim Aushärten (Wärme) der Beschichtung eingebaut wird In der ausgehärteten Beschichtung liegt folglich eine bleibende Hydrophilierung von nur 10 Äquivalent-% seitens des KOH vor.

### Ansatz:

| | | |
|---|---|---|
| 49,5 g | (0,022 Val) | eines monofunktionellen Ethylenoxid-Propylen- |
| | | oxid-Polyethers mit der OH-Zahl 25 |
| 80,0 g | (0,080 Val) | eines Polypropylenglykols mit der OH-Zahl 56 |
| 110,0 g | (0,110 Val) | eines Polycarbonates auf Basis von 1,6-Dihy- |
| | | droxyhexan mit der OH-Zahl 56 |
| 110,0 g | (0,110 Val) | eines auf Trimethylolpropan gestarteten Prop- |
| | | yplenoxyd-Polyethers mit der OH-Zahl 56 |
| 76,5 g | (0,612 g) | 4,4'-Diisocyanato-diphenylmethan |
| 12,18 g | (0,14 Val) | Butanonoxim |
| 60,90 g | (0,15 Val) | Dispergator gemäß Beispiel 4 |
| 14,70 g | (0,14 Val) | 4,4'-Diamino-dicyclohexylmethan |
| 533,57 | | Wasser |
| 1047,35 g | | Dispersion |
| | | Festkörper: 45 % |
| | | Viskosität (23°C): ca. 500 mPa·s |

### Durchführung:

Die obigen Polyole werden bei 100°C 20 mbar entwässert und auf ca. 60°C abgekühlt. Danach gibt man unter Rühren in einem Guß die Gesamtmenge an reinem MDI hinzu, erwärmt langsam auf 70°C und setzt bei dieser Temperatur ca. 3 Stunden um, bis der NCO-Gehalt auf ca. 2,75 % abgesunken ist, berechnet ist ein NCO-Gehalt von 2,86 %. Man verdünnt mit 500 g Aceton und stellt den Ansatz auf ca. 40°C und führt noch eine Kontroll-NCO-Messung durch. Gefunden wird ein NCO-Gehalt von ca. 1,2 %, berechnet sind 1,3 %. Danach gibt man Butanonoxim hinzu, rührt ca. 20 min. bei 40°C und ermittelt einen NCO-Gehalt von ca. 0,58 %, berechnet sind 0,67 %. Anschließend wird die Dispergatorlösung gemäß Beispiel hinzugegeben und ca. 30 min. bei 40°C nachgerührt, bis kein NCO-Gehalt mehr nachweisbar ist. Jetzt wird das Diamin eingerührt und nach ca. 15 min. mit Wasser dispergiert. Nach dem Abdestillieren des Acetons (45°C/100 mbar) erhält man eine milchig blaue Dispersion mit einem Festkörpergehalt von 45 % und einer Viskosität bei 23°C von ca. 500 mPa·s. Die Dispersion ist trotz des potentiell reaktiven Charakters lagerstabil.

### Eigenschaften der Beschichtung aus obiger Dispersion

Die Dispersion wird nach einem bestimmten Trocknungsprogramm (ca. 1 min. 80°C, ca. 1 min. 120°C und ca. 1 min. 140°C) zu einem Film bzw. zu einer Verklebung von PVC mit Polyamid verarbeitet.

### Filmeigenschaften:

| | |
|---|---|
| Modul 100 % | 2,3 MPa |
| Zugfestigkeit tr. | 11,0 MPa |
| Bruchdehnung tr. | 490 % |
| Zugfestigkeit naß | 8,8 MPa |
| Bruchdehnung naß | 510 % |
| Vol. quell EE | 502 % |
| Vol. quell H₂O | 33 % |
| Filmstärke | 70 g/m² |
| | |
| Haftfestkeit auf Polyamid: | |
| trocken (N/2,5 cm) | 34 |
| naß (N/2,5 cm) | 18 |

## Patentansprüche

1. Verwendung von durch Michael-Addition von 1-Amino-3,3,5-trimethyl-5-amino-methyl-cyclohexan mit 1 bis 2 Mol einer α,β-ungesättigten Carbonsäure ausgewählt aus Acrylsäure, Methacrylsäure und Crotonsäure und gegebenenfalls anschließender Neutralisierung mit bis zu der COOH-Gruppe äquivalenten Menge (Erd)alkalihydroxid und/oder Aminen und/oder Ammoniak erhältlichen Stoffen als Hydrophilierungsmittel.

2. Verwendung nach Anspruch 1 zur Herstellung von PUR-Dispersionen.

3. Verwendung nach Anspruch 1 zur Herstellung von Beschichtungsmitteln.

4. Verwendung nach Anspruch 1 zur Beschichtung von Leder und Textilien und Blechen.

## Claims

1. Use of substances obtainable by Michael addition of 1-amino-3,3,5-trimethyl-5-amino-methyl-cyclohexane with 1 to 2 mol of an α,β-unsaturated carboxylic acid chosen from acrylic acid, methacrylic acid and crotonic acid and if appropriate subsequent neutralization with an alkali/alkaline earth metal hydroxide and/or amines and/or ammonia in an amount up to the equivalent of the COOH groups as hydrophilizing agents.

2. Use according to claim 1 for the preparation of PUR dispersions.

3. Use according to claim 1 for the preparation of coating compositions.

4. Use according to claim 1 for coating leather and textiles and sheet metals.

## Revendications

1. Utilisation en tant qu'agents hydrophilisants de substances obtenues par une addition de Michael entre le 1-amino-3,3,5-triméthyl-5-aminométhyl-cyclohexane et une à deux moles d'un acide carboxylique α,β-insaturé choisi parmi l'acide acrylique, l'acide méthacrylique et l'acide crotonique, éventuellement suivie d'une neutralisation par un hydroxyde alcalin ou alcalino-terreux et/ou une amine et/ou l'ammoniac en quantité allant jusqu'à la quantité équivalente par rapport au groupe COOH.

2. Utilisation selon la revendication 1, pour la préparation de dispersions de polyuréthanne.

3. Utilisation selon la revendication 1, pour la préparation de produits de revêtement.

4. Utilisation selon la revendication 1, pour le revêtement du cuir, des matières textiles et des tôles métalliques.
